# EUROPEAN PATENT SPECIFICATION

(11) **EP 4 427 676 B1**
(45) Date of publication and mention of the grant of the patent: **22.10.2025**
(21) Application number: 23382225.3
(22) Date of filing: 09.03.2023
(51) Int. Cl.: A61B 5/268, A61B 5/293, A61B 5/296, A61B 5/00

(54) **SYSTEM FOR ACQUIRING ELECTRICAL SIGNALS FROM A SUBJECT**
SYSTEM ZUR ERFASSUNG ELEKTRISCHER SIGNALE EINER PERSON
SYSTÈME D'ACQUISITION DE SIGNAUX ÉLECTRIQUES D'UN SUJET

(43) Date of publication of application: 11.09.2024
(73) Proprietor: Fundación Tecnalia Research and Innovation, 20009 Donostia (ES)
(72) Inventor: RAMOS MURGUIALDAY, Ander, 20009 Donostia-San Sebastián (ES); BIJELIC, Goran, 20009 Donostia-San Sebastián (ES); ORTEGO ISASA, Iñaki, 20009 Donostia-San Sebastián (ES)
(74) Representative: Hoffmann Eitle

(56) References cited:
- WO-A1-2019/211314
- CN-A- 112 686 158
- US-A1- 2019 192 031
- US-A1- 2022 061 728

## Description

### Technical field of the invention

The present invention belongs to the field of sensors. Particularly, the present invention pertains to systems comprising sensors for acquiring electrical signals for a subject including electroencephalography (EEG), electromyography (EMG) and electrodermal activity, more particularly to a minimally invasive signal recording system.

### Background of the invention

Electrical activity can be measured all along the skin surface of mammal, including the human body. This electrical activity is associated to different processes underlying the skin, which may comprise muscle activation, nerve signals and neural activity among others. Current systems to detect this electrical activity either involve cheap, non-invasive but noisy solutions or expensive, and high quality but invasive solutions. This is the case of EEG recording at the head level.

EEG is the most accessible brain recording system. While state of the art electrodes use silver/silver chloride electrodes for stable interface properties in short time recordings, instability, inconvenience and infections are reported for long-term applications. Therefore, dry electrodes have been under development to overcome these shortcomings and have shown good performance for certain applications where signal to noise ratio and session to session transfer problems are not an issue, but are not yet on the technology readiness level to enter the market as clinical standard. The advent of quantitative EEG displays, such as density spectral array (DSA) and compressed spectral array (CSA), has allowed us to readily detect seizure by colour, instead of waveforms, from long-term EEG data. The subsequent advent of portable digital video EEG systems has enabled easy EEG measurement at any place, including emergency rooms and Intensive Care Units (ICUs). These developments have led to the increased use of EEG in the ICU setting. However, continuous EEG is currently only applied in controlled environments (hospital ICUs).

Unfortunately, the controlled environment of ICUs cannot be found in the everyday environments the patients inhabit, such as their house, the street or their workplace. Therefore, all the advantages of EEG to monitor the brain status in an accessible way cannot be translated to these places, significantly hindering the possibility for a continuous monitoring of brain-related conditions. Having such a system would allow not only to monitor the brain status, but also to detect, diagnose or even prevent events that could have a negative impact on the health of the patients, such as seizures or strokes.

Also, most EEG monitoring systems either 1) provide reliable signals with low noise and high spatial and frequency resolutions at a cost of invasive procedures, such as cortical implants with have limited operational lifespan due to body reaction against the foreign bodies; 2) provide reliable signals with acceptable noise and resolutions but are not removable, leading to foreign bodies staying throughout the whole life of a patient for a short period of time study or 3) unreliable signals with low signal-to-noise ratios (SNR), and low frequency and/or spatial resolutions but that do not require surgical procedures. The latter involve expensive and heavy systems to compensate for the low-quality signals acquired that make them impractical for everyday use.. Current wearable solutions require frequently changing the recording electrodes, which involves attending to an specialist every time they need to be reinstalled. Moreover, each time the electrodes are changes, the location of these electrodes are changed in more or less amount, which hinders the possibility of long-term studies on a subject, and makes the translation of diagnostic analysis techniques from the ICUs to the real life of the subjects. US 2019/0192031 A1 discloses an EEG system which features a sensor implanted behind a user's ear, on or adjacent to their mastoid process. The sensor includes an implanted portion and a connector. The implanted portion includes an electrode that is implanted between the user's skin and skull. A shaft protrudes through user skin, connecting the electrode to a male part of a press stud which is exposed behind the user's ear. The connector attaches a lead to the implanted portion. Generally, the implanted portion of the sensor is sized and shaped for implantation, i.e. the sensor is sufficiently small to fit between the skull and skin.

Other conditions and diseases relating the brain or other parts of the body, such as muscles which also require or would benefit from an out-of-hospital continuous electrical monitoring also face the same problems including complex setup procedures, continuous replacements of registering means or unremovable registering means, low SNR poor quality and low discretion.

There is therefore a need for an electrical signal acquisition system that enables a continuous and unobtrusive recordings in uncontrolled environments, that is portable, can provide reliable recordings, that can be easily removed when necessary and does not require any expertise from the user in order to make it work and for a method thereof.

### Summary of the invention

According to a first aspect of the invention, there is provided a system for acquiring electrical signals from a subject, the system comprising:
a) at least one electrode, the electrode comprising: a support configured to resemble a hair and configured to be partially implanted through hair implant techniques onto the skin surface of a subject at a dermal or subdermal depth through its proximal end, and registering means provided on the proximal end of the support and configured to register an electrical signal associated to an event below the skin surface; and
b) b) a mount configured to be placed over the skin of a subject, the mount comprising: at least one electrically conductive surface on the internal face of the mount; and processing means configured to process the signals obtained through the at least one electrically conductive surface.

The support of the at least one electrode is essentially longitudinal. The at least one electrode further comprises conductive means configured to electrically connect the registering means on the proximal end with the distal end of the support; and the mount is configured to electrically interface with the conductive means at the distal end of the at least one electrode through the at least one electrically conductive surface and process the electrical signal registered by the registering means through the processing means.

In a preferred embodiment of the first aspect of the invention, the registering means is provided on the support by printing or engraving procedures.

In another preferred embodiment, the support comprises synthetic or natural polymers, more preferably Polyethylene glycol (PEG), polyethylene terephthalate, polyester, polyamide, even more preferably Biofibre CE0373.

In another preferred embodiment, at least the minimum number of elements n among the at least one electrode and the at least one electrically conductive surface are configured to be spatially paired one to one, such that each of the n electrically conductive surfaces is placed on the internal face of the mount in the location its paired electrode is configured to be implanted onto the skin.

In another preferred embodiment, the at least one electrode further comprises amplifying means to passively pre-amplify the potential registered by the registering means. In a more preferred embodiment, the amplifying means comprise semiconductive, conductive and insulating layers provided on the support.

In another preferred embodiment, the at least one electrode further comprises an identification element configured to identify the electrode, and wherein the mount further comprises at least one identification means assigned to the at least one electrically conductive surface configured to recognize the identification element.

In the invention, the support of the at least one electrode is configured to resemble a hair.

In another preferred embodiment, the registering means and/or the conducting means of the at least one electrode comprises at least one of the following materials: conductive polymers, carbon-based materials or metal based materials in form of thin films and coatings; preferably wherein the conductive polymers are PEDOT:PSS, Polyaniline (PANI), polyphenylenes, poly(para-phenylenevinylene), Polypyrrole (PPy) or Polythiophenes (PTs) and/or wherein the carbon-based materials are Carbon nanotubes, buckyball structures, Graphene Oxide or Carbon black, and/or wherein the metal-based materials are Ag, AgCl, TiN, Au or Pt.

In another preferred embodiment, the system further comprises a wireless device configured to communicate with the processing means of the mount. In a more preferred embodiment, the wireless device is configured to receive information of the signal quality of the at least one electrically conductive surface from the mount and to determine whether the mount is properly placed over the skin of the subject based on the received information.

In another preferred embodiment, the registering means are configured to register an electrical signal associated to the neural activity of a region of the brain and wherein the mount is a head mount, and the system is for acquiring neural activity signals.

According to a second aspect of the disclosure, there is provided a computer-implemented method for acquiring electrical signals from a subject using a system according to any of the systems of the first aspect of the invention. The method comprises the steps of:
a) providing the at least one electrode at a subdermal depth of the subject's skin,
b) providing the mount over the skin of the subject, and
c) determining that the electrical signal acquired by the mount through at least one electrode is the electrical signal of the subject in the area wherein the at least one electrode is provided.

In a preferred embodiment of the second aspect of the disclosure, the provision of the at least one electrode at a subdermal depth of the subject's skin is performed through hair implant techniques.

In another preferred embodiment, when the at least one electrode (1) further comprises an identification element (18) and the mount (20) further comprises at least one identification means (28), the method further comprises identifying the electrode (10) acquiring a certain electrical signal by recognizing the identification element (18) of said electrode (10) through the identification means (28).

### Brief description of the drawings

To enable a better understanding of the present disclosure, and to show how the present disclosure may be carried out, reference will now be made, by way of example only, to the accompanying schematic drawings, wherein:
Figure 1 shows a diagram of a system for acquiring electrical activity signals according to one or more embodiments of the invention.
Figure 2 shows an schematic view of a system for acquiring electrical activity signals according to one or more embodiments of the invention.
Figure 3 shows an schematic view of an electrode according to one or more embodiments of the invention.
Figure 4 shows diagram of a method for acquiring an electrical activity signal according to one or more embodiments of the invention.

### Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "support" refers to any element that provides a substrate to which other elements of can be attached to or provided onto. In the context of the present invention, the support of the electrode defines the electrode geometrical, mechanical and biocompatibility properties. The term "electrically conductive" refers to an element with the property of allowing the flow of electric current in one or more directions.

The term "subdermal" refers to a depth of any of the skin layers.

### Description

Each embodiment disclosed herein is contemplated as being applicable to each of the other disclosed embodiments. The invention is defined in the appended claims.

As mentioned in the background of the invention, the present invention aims at solving recording problems in many different areas of the human body, such as head, face, arms, legs, chest among other, and is not limited in this sense to any particular area of the body in this regard. It is therefore noted that the present invention, while exemplified in some of the embodiments as a system aimed at the head, in other embodiments the system herein describe can likewise be aimed at other parts of the body without any limitation.

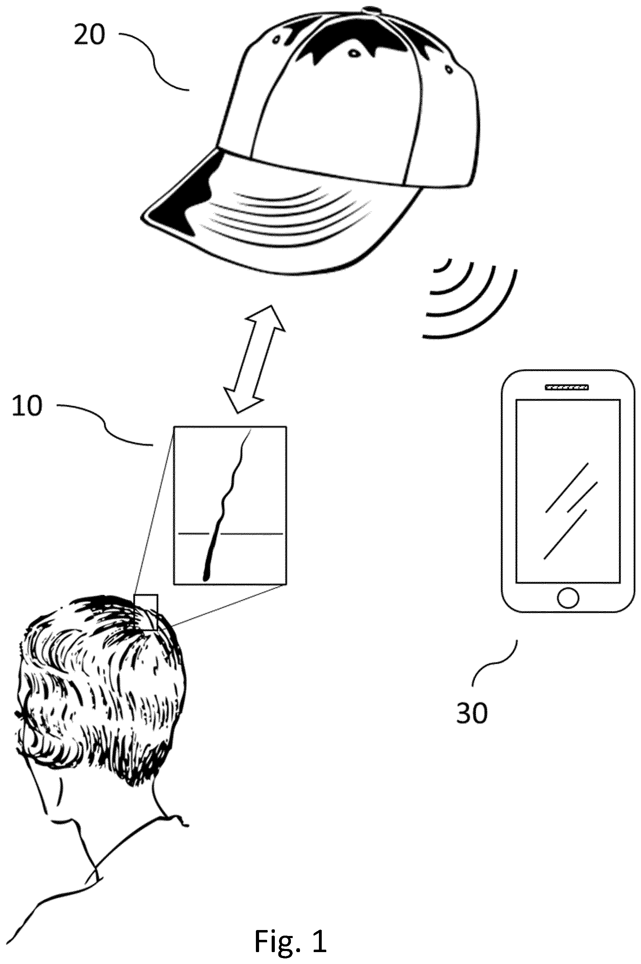

A first aspect of the invention relates to a system for acquiring electrical signals from a subject, as shown with respect to Fig. 1. As explained in the background of the invention, the electrical signals can be registered, and associated to different physiological processes and used for different medical purposes.

The system comprises at least one electrode (10) and a mount (20) configured to be placed over the skin of a subject. It is noted that the mount (20) is shaped in the form of a cap, although in some other embodiments the mount (20) may be in the form of a band, a sleeve, leggings, a glove a face-mask, a cap, a bandana or a head-scurf, among others. It is also noted that Fig. 1 further comprises other optional elements.

As it can be appreciated, Fig. 1 shows a system that comprises many other additional characteristics, that may not be comprised in other embodiments or that may be comprised in different ways. For example, we note that in Fig. 1 there is a wireless device (30) configured to communicate with the mount (20). However, in other embodiments the wireless device (30) may not be comprised within the system, and therefore the mount (20) would not communicate with a wireless device (30).

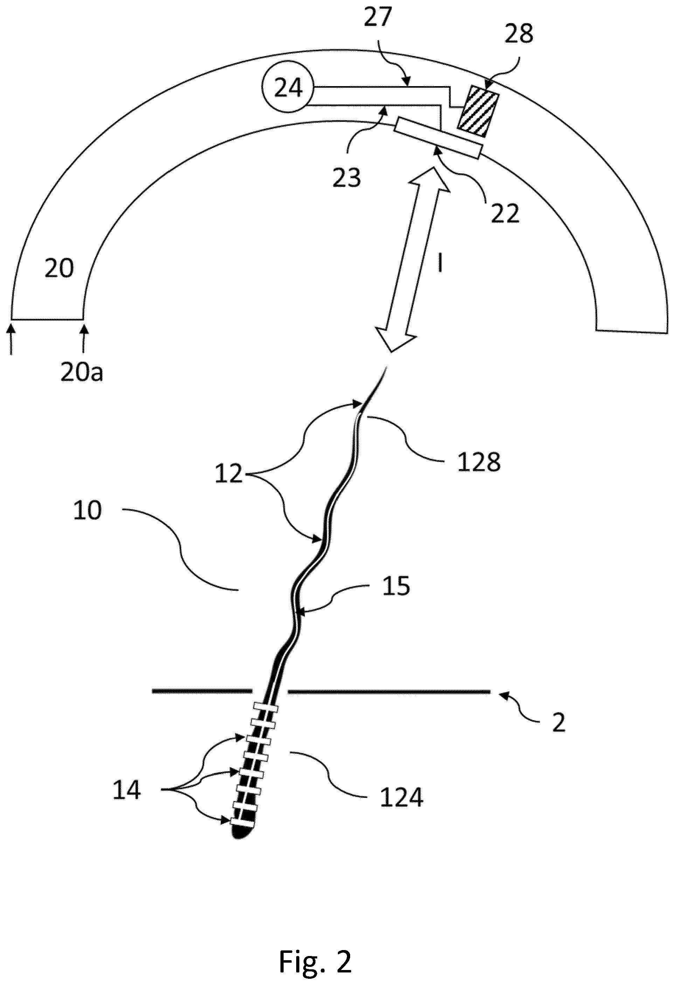

As shown in Fig. 2, the system comprises at least one electrode (10). The electrode (10) of Fig. 2 is not proportional to the mount (20) for clarity purposes. It is understood that the system may comprise, one, two, three, four, five or more than five electrodes (10) according to the present invention. The at least one electrode (10) may have a particular shape, or properties such as certain materials as will be described later on. It is noted that when the system comprises more than one electrode (10), these may be different between them in terms of all the different shapes, materials and properties the at least one electrode (10) may have.

The at least one electrode (10) comprises a support (12). The support (12) is configured to provide the gross shape to the at least one electrode (10) and the structure to which the rest of the elements comprised in the at least one electrode (10) can be attached to. The support (12) is configured to be partially implanted onto the skin surface (2) of a subject at a dermal or subdermal depth. The support (12) is configured to be partially implanted, such that it is configured to have at least a section not implanted onto the skin surface (2). The support (12) may be configured to be partially implanted by different means, for example by having a certain shape or property that enable such implant. For example, the support may have a certain shape that is suitable to be easily implanted but difficult to be removed, such that the implant can be easily implanted. In another example, the support may have a certain shape that is suitable to be easily implanted and explanted. It is noted that the implant is configured to be partially implanted at a dermal or subdermal depth, i.e. it does not require a big surgery to be partially implanted, but it can be done through less invasive methods, such as through a needle configured thereof. Such needle may be for example a tattooing needle or a hair implant needle. It is understood that the support (12) is very preferrable biocompatible, such that it is configured to be partially implanted onto the skin surface (2) of a subject without provoking adverse reactions. The skilled person may appreciate there are many different ways in which a device can be made biocompatible, such as using biocompatible materials or encapsulating the support (12) into a layer comprising a biocompatible material. Many other techniques which may be suitable to make the support (12) biocompatible are also comprised within the present embodiment.

As seen in Fig. 2, the support (12) has a proximal end (124) and a distal end (128) defined. Although in Fig. 2 the proximal end (124) and the distal end (128) are defined in a general way, it is noted that in some embodiments, the proximal end (124) and the distal end (128) may be defined in different ways, for example having a significantly bigger proximal end (124) than distal end (128) or vice versa. The support (12) is configured to be implanted through its proximal end (124).

The at least one electrode (10) also comprises registering means (14) provided on the proximal end (124) of the support (12) and configured to register an electrical signal associated to an event below the skin surface. The registering means (14) can be provided on the support (12) in different ways. For example, the registering means (14) can be provided as a coating over the proximal end (124) of the support (12) or may be printed, imprinted or engraved over the support (12). The skilled person may appreciate there are many other different ways in which the registering means (14) may be provided onto the support (12), all of them comprised within the scope of the present invention. Moreover, the registering means (14) may comprise many different materials suitable to register the electrical signals, such as conductive polymers, carbon-based materials or metal-based materials with a low impedance.

It is noted that there are many different electrical signals associated to an event below the skin surface which may be registered by the registering means (14). For example, the registering means (14) may register the neural activity of a cohort of neurons, the electromyographic activity form a nearby muscle or even the electrodermal activity when an external electrode provides an input current nearby. It is also noted that the registering means, may comprise one or more registering means of the same type, for example, as shown in Fig. 2, the registering means (14) comprises several means at different locations on the proximal end (124) of the support (12). Advantageously, this enables a more robust registering system, as if one of the registering means is disturbed by some noise, the signal can still be properly recorded.

As shown in Fig. 2, the system also comprises a mount (20) configured to be placed over the skin of a subject. As an example, in Figs. 1 and 2 the mount (20) is respectively depicted as a head mount (20) and as a cross section head mount. The mount can take several shapes, such as in the form of a band, a sleeve, leggings, a glove a face-mask, a cap, a bandana or a head-scurf, among others, all of them configured to be placed over the skin of a subject. The skilled person may note that there al multiple variations and alternative in which a mount (20) may be defined, all of them comprised within this embodiment. Moreover, the mount (20) may be configured to be placed over the skin of a subject by different means, such as by having a certain elastic element, like an elastic band, or attaching means like a Velcro-alike closure, or just be designed with a shape configured to attack to a certain part of the skin surface such as the head, either passively, like a hat or actively, like a wearable VR glasses with self-adjustment bands. Thus, the present embodiment is not limited by any means to a certain particular type of mount (20) as long as it comprises the following features that will be now described.

The mount (20) comprises at least one electrically conductive surface (22) on the internal face (20a) of the mount (20). It is therefore understood that the system may comprise, one, two, three, four, five or more than five electrically conductive surfaces (22) according to the present invention. The at least one electrically conductive surface (22) may have a particular shape, thickness, material or properties as will be described later on. The at least one electrically conductive surface (22) may cover the while internal face (20a) on the mount or may just cover a part of the mount (20), in the form of a patch. It is noted that in either case, the at least one electrically conductive surface (22) may take different shapes such as rounded, squared, in the form of a ring, or may have an irregular shape; as well as a more planar or thicker configuration. The at least one electrically conductive surface (22) may be flexible to adapt to the skin surface or may be rigid. It is noted that when the system comprises more than one electrically conductive surface (22), these may be different between them in terms of materials, shape, thickness, configuration and properties the at least one electrically conductive surface (22) may have.

The at least one electrically conductive surface (22) is comprised on the internal face (20a) of the mount (20). The internal face (20a) is the face or surface of the mount (20) that is configured to interface with the skin of the subject, usually in the form of a concave surface, although in some embodiments, it may not be concave. When the mount (20) comprises more than one electrically conductive surfaces (22), these may be disposed over the internal face (20a) of the mount in a certain pattern, or may be each located in different placed in a not clear pattern. For example, for a mount (20) configured to be placed over the head of a subject, the electrically conductive surfaces (22) may be disposed over the internal face (20a) of the mount (20) according to the international 10-20 system for EEG recording. Also symmetry in any axis on the disposition of the more than one electrically conductive surfaces (22) may be preferred. The skilled person may therefore note that there are many way in which more than one electrically conductive surfaces (22) can be comprised in the mount (20). The mount (20) also comprises processing means (24) configured to process the signals obtained through the at least one electrically conductive surface (22). The electrically conductive surface (22) is therefore associated to the processing means (24) through electrically conductive means (23) configured to electrically conduct the signals obtained by the at least one electrically conductive surface (22) towards the processing means (24). In some embodiments, the electrically conductive means (23) may be the electrically conductive surface (22) itself. It is noted that the processing means (24) may comprise one or more processing units, such as a microprocessor, GPU, CPU, multi-core processor or the like.

The processing means (24) may be configured to process the signals obtained through the at least one electrically conductive surface (22) in different ways. For example, the processing means (24) may be configured to filter, amplify, reduce its noise (i.e. increase its SNR) apply hardware or software filters, such as Notch filters low-pass filters, bandpass filters or highpass filters, filter artifacts, spatial and time filtering, process the signal stability and compensate for signal loss, false positive management and detect biomarkers among others. It may also comprise converting the received analogic signals into digital signals The skilled person may appreciate that there are plenty of different ways in which a signal, and particularly a bio signal may be processed to obtain the most of it, all of them comprised within the processing configuration of the processing means (24) according to the present invention.

It is noted, that in some embodiments, the processing means (24) may make use of advanced processing solutions such as machine learning (ML) and/or artificial intelligence (AI) in order to process the signals obtained through the at least one electrically conductive surface (22). For example, ML and/or Al solutions integrated into the processing means (24) may enable the filtering or noise removal abovementioned in real time. ML and/or Al may also enable for a better understanding of neurological diseases, therefore allowing to design predictive and monitoring self-optimizing models.

As shown in Fig. 2 the support (12) of the at least one electrode (10) is essentially longitudinal. The axis defining this longitudinal shape is defined by the proximal end (122) and the distal end (124) of the support (12). However, it is noted that the support may still not be lineal and have certain curves on its shape. It is also noted that the support may be flexible and thus it may not always be in placed in a longitudinal shape as shown in Fig. 2.

The at least one electrode (10) further comprises electrically conductive means (15) configured to electrically connect the registering means (14) on the proximal end (124) with the distal end (128) of the support (12) of the at least one electrode (10). This allows the signal acquired at the proximal end (124) to be transferred to the distal end (128) of the electrode. As the support (12) is configured to be partially implanted onto the skin surface (2), the electrically conductive means (15) of the at least one electrode (10) allows thew signal acquired at a dermal or subdermal depth to be transferred to the exterior of the skin at the distal end (128). The electrically conductive means (15) may comprise for example a wire or a conductive path imprinted over or within the support (12). The skilled person may envisage other electrically conductive means (15) at the light of the present invention and the general common knowledge which are also comprised within this invention.

Also, as shown by arrow I, the mount (20) is configured to electrically interface with the electrically conductive means (15) at the distal end (128) of the at least one electrode (10) through the at least one electrically conductive surface (22) and process the electrical signal registered by the registering means (14) through the processing means (24). Thus, when the mount (20) is placed over the skin of the subject, the electrically conductive surface (22) will enter in contact with the electrically conductive means (15) at the distal end (128) of the support (12) of the at least one electrode (10), and thus, the signals acquired at the proximal end (124) of the support (12) can be effective transferred through the registering means (14) along the electrically conductive means (15) to the distal end (128), and from there to the at least one electrically conductive surface (22) which as above mentioned is connected to the processing means, wherein the signal is processed.

Thus, it is understood that the at least one electrically conductive surface (22) is configured to be placed on the internal face (20a) of the mount (20) at the locations at which the at least one electrode (10) will be partially implanted onto the skin surface (2). The locations may be defined according to a particular location system, for example the International 10-20 system when the mount (20) is a head mount (20).

Advantageously, the present invention provides an electrical signal acquisition system that enables a continuous and unobtrusive recordings in uncontrolled environments, that is portable, easily removable and can provide reliable recordings, thanks to the partially implanted electrodes (10) at a dermal or subdermal depth that can be hindered in appearance among the hair of the subject as be removed from its distal end when necessary, and wherein the mount (20) processing the signals to a scientifically usable level is disguised as a regular mount, such as a hat, a mask or an armband.

As it can be appreciated, Fig. 2 shows a system that comprises many other additional characteristics, that may not be comprised in other embodiments or that may be comprised in different ways. For example, we note that in Fig. 2 the mount (20) is represented in a schematic way in form of a semicircle. However, in other embodiments, the mount (20) may have different shapes as above mentioned, in form of a facemask, an armband, a hat, a cap, a bandana or a head-scurf. It is also noted that the mount (20) may not have such a curved shape, but it may have a more planar configuration or may be completely circular depending on the view. In this sense the mount (20) of Fig. 2 is only representing what a cut-view of a head mount (20) could look like. It is also noted that the electrode (10) in Fig. 2 has a shape that resembles a hair. However, in some embodiments, the electrode may not be shapes as a hair, and be just essentially longitudinal. In some other embodiments, the electrode may be shaped in other forms of hair, such as curly hair. It is also noted that the registering means (14) in Fig. 2 is represented as a complex, multi-levelled system of elements. However, it is noted that in other embodiments, the registering means (14) may not comprise such structure but rather have another shape, and may be for example a layer coating the support (12), or may be partially embedded within the support (12). It is therefore noted that the registering means (14) of Fig. 2 is just one of many ways in which an registering means (14) can be provided on the support (12).

Additionally, it is also noted that the processing means (24) of Fig. 2 is schematically represented as a circle but it can comprise several integrated circuits (IC). It may also be alternatively comprised within on the internal face (20a) and/or the external face (20b) of the mount (20) for example by designing the circuits on any of the surfaces through CMOS technology. This may be true for all or some of the ICs within the processing means. It is also noted that the electrically conducting means (23) are shown in Fig. 2 in a schematic way as a single line, although in some embodiments the electrically conducting means (23) may comprise more than one electrically conducting tracks. It is also noted that the at least one electrically conductive surface (22) is schematically shown as a rectangle on the internal face (20a) of the mount (20), however, in other embodiments the at least one electrically conductive surface (22) may take different shapes, as above-mentioned. Lastly, it is noted that the mount (20) of Fig. 2 comprises one identification means (28), however, in other embodiments of the invention the mount may not comprise such identification means (28). At the light of this description, the skilled person can foresee this and many other alternatives to make the at least one electrode (10), the registering means (14), the mount (20), the at least one electrically conductive surface (22) the electrically conducting means (23) and the processing means (24).

As above mentioned, the electrode (10) of Fig. 2 is shown out of proportion, much bigger than the mount (20) for clarity purposes. It is also noted that the electrode (10) is shown partially introduced into a skin surface (2) although in other embodiments, the at least one electrode (10) is not provided necessarily within a skin surface, but the at least one electrode (10) is just configured to be partially implanted onto the skin surface (2) of a subject.

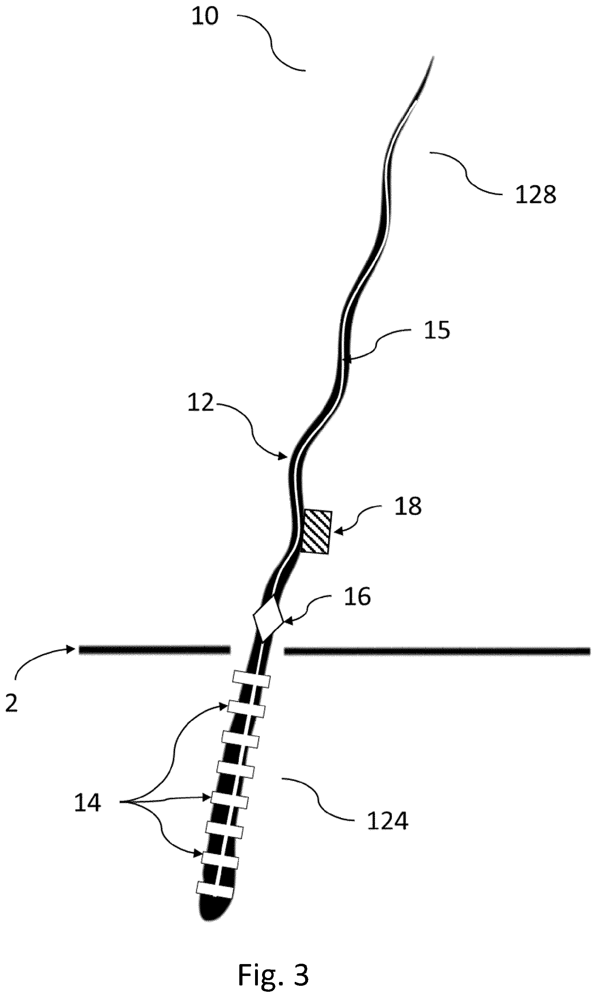

Fig. 3 shows a schematic view of an electrode (10) according to one or more embodiments of the invention. As it can be appreciated that as in Fig. 2, Fig. 3 shows an electrode (10) that comprises many other additional characteristics, that may not be comprised in other embodiments or that may be comprised in different ways. Those in common such as the shape of the electrode (10), the representation of the registering means (14) and the electrode (10) being represented partially introduced into a skin surface (2) can be applied likewise. It can be also noted that in Fig. 3 the registering means (14) extends along the support (12) such that it is configured to electrically conduct the proximal end (124) and the distal end (128) of the support (12). It also it comprises additional optional elements, such as amplifying means (16) and identification means (18), as will be explained later in some preferred embodiments.

In a preferred embodiment of the first aspect of the invention, the registering means (14) is provided on the support (12) by printing or engraving procedures. The skilled person may note there are many techniques in the state of the art for providing means configured to register electrical signals by printing procedures on a surface like the support (12) as shown for example in Velcescu et al. (Flexible 3D-printed EEG electrodes. Sensors. 2019 Apr 6;19(7):1650) and in Krachunov et al (3D printed dry EEG electrodes. Sensors. 2016 Oct 2;16(10):1635). Thus, the registering means (14) can effectively be provided onto the support (12).

Advantageously, the registering means (14) is provided on the support (12) of the electrode, such that it does not essentially modify the properties of the support (12) such as the geometrical and/or mechanical and/or biocompatibility properties of the same.

In another preferred embodiment, the support (12) comprises at least one of the following materials: synthetic or natural polymers, more preferably Polyethylene glycol (PEG), polyethylene terephthalate, polyester, polyamide, even more preferably Biofibre CE0373.

Advantageously, this allows for the support (12) to be biocompatible, as defined in different standardization protocols such as the biocompatibility testing of ISO 10993, taking not only cytotoxicity questions into account but also systemic compatibility, allergenic reactions, eluates and degradation stability of the prototypes under the intended use case.

In another preferred embodiment, at least the minimum number of elements n among the at least one electrode (10) and the at least one electrically conductive surface (22) are configured to be spatially paired one to one. Thus, each of the electrically conductive surfaces (22) is placed on the internal face (20a) of the mount (20) in the location it's paired electrode (10) is configured to be partially implanted onto the skin surface (2).

In practice this means that when the system comprises at least one electrode (10) and at least one electrically conductive surface (22) with any number of elements each, the minimum number of elements among each the at least one electrode (10) and the at least one electrically conductive surface (22) are spatially paired one to one. Thus given a system comprising x electrodes (10) and y electrically conductive surfaces (22) wherein x<y, n=x electrodes (10) will be spatially paired one-to-one with "n" electrically conductive surfaces (22); and if x>y, n=y electrodes (10) will be spatially paired one-to-one with n electrically conductive surfaces (22) for any x and y elements.

In order to spatially pair each of the paired electrodes (10) and each of the paired electrically conductive surfaces (22), the location of the each of the at least one electrically conductive surface (22) is conditioned by the location on the surface (2) its paired electrode is configured to be partially implanted on. Thus, each of the at least one electrode (10) has been assigned a location on the skin surface (2). This may be done for example to a coordinate system, such as the International 10-20 system.

It is noted than in some other embodiments of this preferred embodiment, the number of electrodes (10) may be greater than the number of electrically conductive surfaces (22) or vice versa, which could be used to add redundancy to the measurements which can further improve the readings. Particularly, when the number of electrically conductive surfaces (22) is greater than the number of electrodes (10) this increases the resilience of the electrical interface between them, as if one electrically conductive surfaces (22) does not contact temporarily with an electrode, other electrode may do so, ensuring that the signal is not lost.

Advantageously, this configures the system to be able to register information on different locations of the skin surface simultaneously.

According to another preferred embodiment, the system comprises more than one electrode (10) configured to be implanted into the same location of the skin surface (2). When more than one electrode (10) are implanted into the same particular area, preferably really close to each other, the same electrically conductive surface (22) of the mount (22) may be in contact with the distal part (128) of the different electrodes (10). Advantageously, this provides redundancy in the electrical interface between the electrode (10) and the mount (20), so that if one electrode (10) does not contact the electrically conductive surface (22) , the electrical signals can still be acquired, as other electrodes (10) on the same area still interface with the same electrically conductive surface (22).

According to another preferred embodiment, the system comprises more than one electrode (10) configured to be implanted into different locations of the skin surface (2). The electrodes may be configured to be placed on a similar location one close to the other or in a certain pattern, such as in a matrix configuration. Advantageously, this further enables complex spatial interpretations of the signals acquired by the registering means (14) of each electrode (10). These complex spatial interpretations can enable the creation of spatial filters for the acquired electrical signal once the signals are processed by the processing means (24), and may for example comprise an average reference filter, or a Laplacian or centre surround filter with floating members depending on for example the impedance check or the signal quality (good, bad or even broken).

According to another preferred embodiment, the at least one electrode (10) further comprises amplifying means (16) to passively pre-amplify the potential registered by the registering means (14). The skilled person may be aware of different ways in which the amplifying means (16) may be comprised in the at least one electrode (10) for example as described in Shad EH et al. (Impedance and noise of passive and active dry EEG electrodes: a review. IEEE Sensors Journal. 2020 Jul 27;20(24):14565-77) and in Xu J et al. (Active electrodes for wearable EEG acquisition: Review and electronics design methodology. IEEE reviews in biomedical engineering. 2017 Jan 20;10:187-98). The amplifying means may be comprised between the proximal end and the distal end of the registering means (14) over the support (12), such that the signals acquired at the proximal end are amplified before reaching the distal end (128) of the support (12), for example on the electrically conductive means (15). At the distal end, the electrode (10) interfaces with the at least one electrically conductive surface (22) of the mount (20) for its processing by the processing means (24). For example, the amplifying means may comprise DC-coupled amplifiers that employ chopping to reduce the acquired noise. It is noted that such amplifying means may be wirelessly powered through an electromagnetic field provided by a device on the mount (20), that may be associated to the at least one identification means (28) as will be described below.

Advantageously, this configures the electrode (10) to provide a cleaner signal towards the mount (20) by attenuating different noise sources coming from motion artifacts, thermal artifacts flicker, or line noise among others, which facilitates the further processing of the acquired neural signals on later stages, such as by the processing means (24) of the mount (20) which in turn improves the performance of the acquisition system.

According to a more preferred embodiment, the amplifying means (16) comprise semiconductive, conductive and insulating layers provided on the support (12).

In another preferred embodiment as shown with respect to Fig. 2, the at least one electrode (10) further comprises an identification element (18) configured to identify the electrode, and the mount (20) further comprises at least one identification means (28) assigned to the at least one electrically conductive surface (22) configured to recognize the identification element (18).

It is noted that the identification element (18) and the at least one identification means (28) are schematically represented in Figs. 2 and 3 but they can comprise several integrated circuits (IC). The at least one identification means (28) may also be alternatively comprised within on the internal face (20a) and/or the external face (20b) of the mount (20) for example by designing the circuits on any of the surfaces through CMOS technology. This may be true for all or some of the ICs within the identification means (28).

Preferably, the at least one identification means (28) are assigned to the at least one electrically conductive surface (22) in a one-to-one basis, such that there are as many identification means (28) as electrically conductive surfaces (22).

The identification element (18) may comprise any technology suitable for identifying a device. For example, the identification element (18) may comprise an RFID tag, a Bluetooth passive transceiver, a Zigbee passive transceiver or an NFC tag . Likewise, the at least one identification means (28) of the mount (20) may comprise any equivalent technology suitable for reading the identification element (18) such as a RFID reader, a Bluetooth receiver, a Zigbee transceiver or an NFC tag. Moreover, as shown in Fig. 2 in some embodiments of this preferred embodiment the at least one identification means (28) is electrically connected to the processing means (24) through electrically conductive means (27) configured to electrically conduct the identification information obtained towards the processing means (24). However, it is noted that in some other embodiments, the electrically conductive means (27) may not be present, and the at least one identification means (28) may be connected to the processing means (24) through any wireless communication protocol such as Bluetooth, Bluetooth Low Energy (BLE), NFC. Zigbee RFID or Wi-Fi.

Advantageously, when the at least one electrode (10) further comprises an identification element (18) and the mount further comprises at least one identification means (28) allows for the processing means (24) to be able to determine the electrode (10) the at least one electrically conductive surface (22) is interfacing with, which can be useful to discriminate between electrodes when two of them are too close. This can further help to determine if the mount (20) has been appropriately placed over the skin of a subject.

In another preferred embodiment as shown in any of Figs. 1, 2 or 3, the support (12) of the at least one electrode (10) is further configured to resemble a hair. It is noted that hairs have different shapes such as straight or curly, colours varying from black to white and thickness. Thus according to this preferred embodiment, the support (12) of the at least one electrode (10) may have any of the possible characteristics of a hair. It is also noted that when the system comprises more than one electrode (10), all the supports (12) of the different electrodes (10) may have the same characteristics, or may vary. In this sense, the support (12) is preferably configured to resemble a hair of the subject to which the electrode (10) is configured to be partially implanted on its skin surface (2) of the head.

Advantageously, having the support (12) further configured to resemble a hair makes the electrode (10) more natural, helps to disguise the electrode when not in use, i.e. when the mount is not placed over it. This has a huge effect on the moral, and social acceptance for patients outside the ICU and hospitals in their daily lives

In another preferred embodiment, the registering means (14) and/or the conducting means (15) of the at least one electrode (10) comprises at least one of the following materials: conductive polymers, carbon-based materials or metal-based materials in form of thin films and coatings. In a more preferred embodiment, the conductive polymers are PEDOT:PSS, Polyaniline (PANI), polyphenylenes, poly(para-phenylenevinylene), Polypyrrole (PPy) or Polythiophenes (PTs) and/or wherein the carbon-based materials are Carbon nanotubes, buckyball structures, Graphene Oxide or Carbon black, and/or wherein the metal-based materials are Ag, AgCl, TiN, Au or Pt. Nevertheless it is noted that the skilled person may envisage other metals and alloys as known in the state of the art that could be also comprised in the registering means (14) and/or the conducting means (15) of the at least one electrode (10), and are therefore also comprised within the present embodiment. Advantageously, this configures the registering means (14) and/or the conducting means to reduce the impedance of the transmission of the electrical signal acquired.

In another preferred embodiment as shown in Fig. 1, the system further comprises a wireless device (30) configured to communicate with the processing means (24) of the mount (20). The wireless device may be any device capable of communicating through a wireless communication protocol such as Bluetooth, Bluetooth Low Energy (BLE), NFC. Zigbee RFID or Wi-Fi with another device. For example the wireless device (30) may be a server, a computer, a smartphone, a tablet or any other wearable. The wireless device preferably comprises feedback means for the user such as a screen a speaker, a led or a haptic motor. The skilled person may envisage may other feedback elements any of these wireless device may comprise, all of them comprised within this preferred embodiment. Preferably, the wireless device will operate in the Industrial, Scientific and Medical (ISM) bands authorised by the International Telecommunication Union (ITU) for such kind of application, for example the 13,56 MHz and/or the 2.4 GHz bands.

Advantageously this allows to make the mount (20) more practical as it can be connected to further means (30) which expands the data processing and/or feedback information to the patient and/or to the clinicians. This might enable computing capabilities for out of ICU data analysis.

In a more preferred embodiment, the wireless device (30) is configured to receive information of the signal quality of the at least one electrically conductive surface (22) from the mount (20) and to determine whether the mount (20) is properly placed over the skin of the subject based on the received information.

The signal quality of the at least one electrically conductive surface (22) from the mount (20) may be obtained by different ways the skilled person may note. For example, the signal-to-noise ratio (SNR) can be used to determine the signal quality of the at least one electrically conductive surface (22). Alternatively, the amplitude or the steadiness of the signal can be used to determine its signal quality. If the signal is not strong enough or if its constantly being interrupted, this may be a sign that the connection with the at least one electrode may not be good enough. These and all the other different solutions the skilled person may derive from the present description are likewise comprised within this more preferred embodiment.

It can be determined whether the mount (20) is properly placed over the skin of the subject based on the information of the signal quality, as if the signal quality is not good enough, it can be determined that the mount (20) is not correctly placed over the skin surface. Moreover, when the system comprises a plurality of electrically conductive surfaces (22), the information may comprise information of the signal quality of the different electrically conductive surfaces (22), which could provide further information on which part of the mount (20) is properly placed over the skin of the subject and which part is not properly placed over the skin of the subject.

Advantageously, having the wireless device (30) configured to receive information of the signal quality of the at least one electrically conductive surface (22) and to determine whether the mount (20) is properly placed over the skin of the subject based on the received information, allows the patient /user to properly self-manage the system adjustment in a simple way without requiring further technical knowledge. For example, the wireless device (30) may inform the subject through feedback elements on which parts of the mount (20) to readjust. Effectively, this enables the use of the system for acquiring neural activity signals from a subject in everyday life without requiring any expertise from the user in order to make it properly work.

In another preferred embodiment of the first aspect of the invention as shown in Fig. 2, the system is for acquiring neural activity signals. Thus, the registering means (14) are configured to register an electrical signal associated to the neural activity of a region of the brain and the mount (20) is a head mount (2). It is therefore understood that the support (12) of the at least one electrode is very much preferably configured to be partially implanted onto the skin surface (2) of the head of a subject.

When the support (12) is configured to be partially implanted onto the skin surface (2) of the head, the registering means (14) can register the reflects the summation of the synchronous activity of thousands or millions of neurons that have similar spatial orientation, represented as a single action potential or potential as electrically conductive materials can register these action potentials (AP's) as a signal. Therefore, by providing this registering means (14) onto the support (12), the electrode (10) is essentially defined itself, with the capacity to capture the neural activity signals of nearby neurons, such as pyramidal neurons of the cortex closer most to the electrode (10).

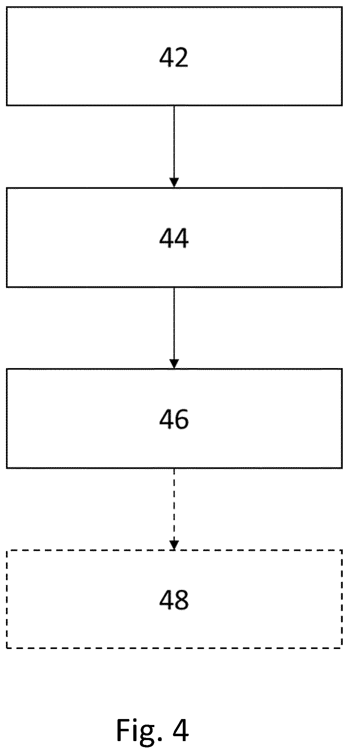

A second aspect of the disclosure refers to a method for acquiring a neural activity signal from a subject using a system according to any of the systems of the first aspect of the invention. As shown in Fig. 4 the method comprises the following steps.

A first step 402, involves providing the at least one electrode (10) at a dermal or subdermal depth of the subject's skin (2). In order to provide the at least one electrode (10) at a dermal or subdermal depth, many techniques may be employed . For example, the at least one electrode (10) can be partially implanted according to a hair implant procedure, wherein the hair or hair graft to be partially implanted is substituted by the at least one electrode (10). Alternatively, the at least one electrode (10) can be partially implanted using a computer-assisted needle implantation system, or by directly poking the at least one electrode (10) onto the skin surface. In another example, the at least one electrode (10) can be partially implanted according to a tattooing technique. It is noted that when the at least one electrode (10) comprises more than one electrode (10) according to any of the electrodes (10) of the first aspect of the invention, they may be partially implanted in the same graft or in different locations and using the same or different techniques.

Then, in step 404 the method comprises providing the mount (20) over the skin surface of the subject. This may be performed by a specialist, or by the very same subject. As the mount is disguised as a regular wearable element, the procedure is simplified. When the system further comprises a wireless device (30) and the wireless device is configured to receive information of the signal quality of the at least one electrically conductive surface (22) of the mount (20), the subject can provide the mount (20) over its own skin without any further help, as the wireless device (30) can determine whether the mount (20) is properly placed over the skin of the subject based on the received information. Thus, through feedback means, the wireless device (30) can guide the inexperienced subject.

Finally, the method comprises the step 406 of determining that the electrical signal acquired by the mount (20) through at least one electrode (10) is the neural activity signal of the subject in the area wherein the at least one electrode (10) is provided. To do so, the signal may be amplified or prefiltered to extract the neural signal form the unwanted noise. Advantageously, this allows for a method for acquiring a neural activity signal in a continuous and minimally intrusive way in uncontrolled environments, that does not require any expertise from the user in order to make it work.

In a preferred embodiment when the system comprises more than one electrode (10) in a certain region and/or when an electrode (10) comprises multiple one registering means (10), the method further comprises improving the SNR by combining (e.g. spatial filtering by averaging) the plurality of signals received form the plurality of electrodes (10).

In another preferred embodiment, the provision of the at least one electrode (10) at a dermal or subdermal depth of the subject's skin (2) is performed through hair implant techniques. Therefore, the at least one electrode (10) may be provided at a dermal or subdermal depth through an implanter configured to open micro-channels on the skin surface at the same time the at least one electrode (10) is provided.

Advantageously, this allows for a simple and safe way to provide the at least one electrode (10) at a dermal or subdermal depth of the subject's skin (2).

In another preferred embodiment, when the at least one electrode (1) further comprises an identification element (18) and the mount further comprises at least one identification means (28), the method further comprises identifying the electrode (10) acquiring a certain electrical signal by recognizing the identification element (18) of said electrode (10) through the identification means (28). Advantageously, this further allows to determine whether the mount (20) is properly placed over the skin of the subject based on the received information. All of the above are fully within the scope of the present disclosure and are considered to form the basis for alternative embodiments in which one or more combinations of the above-described features are applied, without limitation to the specific combination disclosed above. In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## Claims

1. A system for acquiring electrical signals from a subject, the system comprising:
a. at least one electrode (10), the electrode comprising:
i. a support (12) configured to resemble a hair and configured to be partially implanted through hair implant techniques onto the skin surface (2) of a subject at a dermal or subdermal depth through its proximal end (124), and
ii. registering means (14) provided on the proximal end (124) of the support (12) and configured to register an electrical signal associated to an event below the skin surface; and
b. a mount (20) configured to be placed over the skin of a subject, the mount (20) comprising:
i. at least one electrically conductive surface (22) on the internal face (20a) of the mount (20); and
ii. processing means (24) configured to process the signals obtained through the at least one electrically conductive surface (22);
wherein the support (12) of the at least one electrode (10) is essentially longitudinal,
wherein the at least one electrode (10) further comprises conductive means (15) configured to electrically connect the registering means (14) on the proximal end (124) with the distal end (128) of the support (12); and
wherein the mount (20) is configured to electrically interface with the conductive means (15) at the distal end (128) of the at least one electrode (10) through the at least one electrically conductive surface (22) and process the electrical signal registered by the registering means (14) through the processing means (24).

2. The system according to claim 1, wherein the registering means (14) is provided on the support (12) by printing or engraving procedures.

3. The system according to any of claims 1 or 2, wherein the support (12) comprises synthetic or natural polymers.

4. The system according to claim 3, wherein the support (12) comprises Polyethylene glycol (PEG), polyethylene terephthalate, polyester, polyamide.

5. The system according to claim 4, wherein the support (12) comprises Biofibre CE0373.

6. The system according to any of claims 1 to 5, wherein at least the minimum number of elements n among the at least one electrode (10) and the at least one electrically conductive surface (22) are configured to be spatially paired one to one, such that each of the n electrically conductive surfaces (22) is placed on the internal face (20a) of the mount (20) in the location its paired electrode (10) is configured to be implanted onto the skin (2).

7. The system according to any of claims 1 to 6, wherein the at least one electrode (10) further comprises amplifying means (16) to passively pre-amplify the potential registered by the registering means (14).

8. The system according to claim 7, wherein the amplifying means (16) comprise semiconductive, conductive and insulating layers provided on the support (12).

9. The system according to any of claims 1 to 8, wherein the at least one electrode (10) further comprises an identification element (18) configured to identify the electrode, and wherein the mount (20) further comprises at least one identification means (28) assigned to the at least one electrically conductive surface (22) configured to recognize the identification element (18).

10. The system according to any of claims 1 to 9, wherein the registering means (14) and/or the conducting means (15) of the at least one electrode (10) comprises at least one of the following materials: conductive polymers, carbon-based materials or metal based materials in form of thin films and coatings.

11. The system according to claim 10, wherein the conductive polymers are PEDOT:PSS, Polyaniline (PANI), polyphenylenes, poly(para-phenylenevinylene), Polypyrrole (PPy) or Polythiophenes (PTs) and/or wherein the carbon-based materials are Carbon nanotubes, buckyball structures, Graphene Oxide or Carbon black, and/or wherein the metal-based materials are Ag, AgCl, TiN, Au or Pt.

12. The system according to any of claims 1 to 11, wherein the system further comprises a wireless device (30) configured to communicate with the processing means (24) of the mount (20).

13. The system according to claim 12, wherein the wireless device (30) is configured to receive information of the signal quality of the at least one electrically conductive surface (22) from the mount (20) and to determine whether the mount (20) is properly placed over the skin of the subject based on the received information.

14. The system according to any of claims 1 to 13, wherein the registering means (14) are configured to register an electrical signal associated to the neural activity of a region of the brain and wherein the mount (20) is a head mount and the system is for acquiring neural activity signals.

## Patentansprüche

1. System zur Erfassung elektrischer Signale einer Person, wobei das System Folgendes umfasst:
a. mindestens eine Elektrode (10), wobei die Elektrode Folgendes umfasst:
i. eine Stütze (12), welche dazu ausgebildet ist, einem Haar zu ähneln, und dazu ausgebildet ist, über Haarimplantationstechniken auf der Hautoberfläche (2) einer Person an einer kutaner oder subkutaner Tiefe über deren proximales Ende (124) teilweise implantiert zu werden, und
ii. Aufzeichnungsmittel (14), welche auf dem proximalen Ende (124) der Stütze (12) bereitgestellt sind und dazu ausgebildet sind, ein elektrisches Signal, welches mit einem Ereignis unter der Hautoberfläche assoziiert ist, aufzuzeichnen; und
b. eine Halterung (20), welche dazu ausgebildet ist, über der Haut einer Person platziert zu werden, wobei die Halterung (20) Folgendes umfasst:
i. mindestens eine elektrisch leitende Oberfläche (22) auf der Innenfläche (20a) der Halterung (20); und
ii. Verarbeitungsmittel (24), welche dazu ausgebildet sind, die über die mindestens eine elektrisch leitende Oberfläche (22) erhaltenen Signale zu verarbeiten;
wobei die Stütze (12) der mindestens einen Elektrode (10) im Wesentlichen länglich ist,
wobei die mindestens eine Elektrode (10) zusätzlich leitende Mittel (15) umfasst, welche dazu ausgebildet sind, die Aufzeichnungsmittel (14) auf dem proximalen Ende (124) mit dem distalen Ende (128) der Stütze (12) elektrisch zu verbinden; und
wobei die Halterung (20) dazu ausgebildet ist, sich mit den leitenden Mitteln (15) am distalen Ende (128) der mindestens einen Elektrode (10) über die mindestens eine elektrisch leitende Oberfläche (22) elektrisch zu koppeln und das elektrische, von den Aufzeichnungsmitteln (14) aufgezeichnetes Signal über die Verarbeitungsmittel (24) zu verarbeiten.

2. System nach Anspruch 1, wobei die Aufzeichnungsmittel (14) auf der Stütze (12) mittels Druck- oder Graviermethoden bereitgestellt sind.

3. System nach einem der Ansprüche 1 oder 2, wobei die Stütze (12) synthetische oder natürliche Polymere umfasst.

4. System nach Anspruch 3, wobei die Stütze (12) Polyethylenglykol (PEG), Polyethylenterephthalat, Polyester oder Polyamid umfasst.

5. System nach Anspruch 4, wobei die Stütze (12) Biofibre CE0373 umfasst.

6. System nach einem der Ansprüche 1 bis 5, wobei mindestens die Mindestzahl von Elementen n unter der mindestens einen Elektrode (10) und der mindestens einen elektrisch leitenden Oberfläche (22) dazu ausgebildet sind, räumlich eins-zu-eins gepaart zu werden, sodass jede der n elektrisch leitenden Oberflächen (22) auf der Innenfläche (20a) der Halterung (20) am Ort, in welchem deren gepaarte Elektrode (10) dazu ausgebildet ist, auf der Haut (2) implantiert zu werden, platziert wird.

7. System nach einem der Ansprüche 1 bis 6, wobei die mindestens eine Elektrode (10) zusätzlich Verstärkungsmittel (16) umfasst, um das von den Aufzeichnungsmitteln (14) aufgezeichnete Potential passiv vorzuverstärken.

8. System nach Anspruch 7, wobei die Verstärkungsmittel (16) halbleitende, leitende und isolierende Schichten umfassen, welche auf der Stütze (12) bereitgestellt werden.

9. System nach einem der Ansprüche 1 bis 8, wobei die mindestens eine Elektrode (10) zusätzlich ein Identifikationselement (18) umfasst, welches dazu ausgebildet ist, die Elektrode zu identifizieren, und wobei die Halterung (20) zusätzlich mindestens ein der mindestens einen elektrisch leitenden Oberfläche (22) zugeordnetes Identifikationsmittel (28) umfasst, welches dazu ausgebildet ist, das Identifikationselement (18) zu erkennen.

10. System nach einem der Ansprüche 1 bis 9, wobei die Aufzeichnungsmittel (14) und/oder die leitenden Mittel (15) der mindestens einen Elektrode (10) mindestens eins der folgenden Materialien umfassen: leitende Polymere, Materialien auf Kohlenstoffbasis oder Materialien auf Metallbasis in Form von dünnen Filmen und Beschichtungen.

11. System nach Anspruch 10, wobei die leitenden Polymere PEDOT:PSS, Polyanilin (PANI), Polyphenylene, Poly(para-phenylen-vinylen), Polypyrrol (PPy) oder Polythiophene (PTs) sind und/oder wobei die Materialien auf Kohlenstoffbasis Kohlenstoffnanoröhrchen, Buckyball-Strukturen, Graphenoxid oder Ruß sind, und/oder wobei die Materialien auf Metallbasis Ag, AgCl, TiN, Au oder Pt sind.

12. System nach einem der Ansprüche 1 bis 11, wobei das System zusätzlich eine drahtlose Vorrichtung (30) umfasst, welche dazu ausgebildet ist, mit den Verarbeitungsmitteln (24) der Halterung (20) zu kommunizieren.

13. System nach Anspruch 12, wobei die drahtlose Vorrichtung (30) dazu ausgebildet ist, Information über die Signalqualität der mindestens einen elektrisch leitenden Oberfläche (22) von der Halterung (20) zu empfangen und basierend auf der empfangenen Information zu bestimmen, ob die Halterung (20) ordnungsgemäß über der Haut der Person platziert ist.

14. System nach einem der Ansprüche 1 bis 13, wobei die Aufzeichnungsmittel (14) dazu ausgebildet sind, ein elektrisches Signal, welches mit der neuralen Aktivität eines Bereichs es Hirns assoziiert ist, aufzuzeichnen, und wobei die Halterung (20) eine Kopfhalterung ist und das System zur Erfassung von neuralen Aktivitätssignalen ist.

## Revendications

1. Système d'acquisition de signaux électriques d'un sujet, le système comprenant :
a. au moins une électrode (10), l'électrode comprenant :
i. un support (12) configuré pour ressembler à un cheveu et configuré pour être partiellement implanté par des techniques d'implantation de cheveux sur la surface de la peau (2) d'un sujet à une profondeur dermique ou sous-dermique à travers son extrémité proximale (124), et
ii. des moyens d'enregistrement (14) prévus sur l'extrémité proximale (124) du support (12) et configurés pour enregistrer un signal électrique associé à un événement sous la surface de la peau ; et
b. une monture (20) configurée pour être placée sur la peau d'un sujet, la monture (20) comprenant :
i. au moins une surface électriquement conductrice (22) sur la face interne (20a) de la monture (20) ; et
ii. des moyens de traitement (24) configurés pour traiter les signaux obtenus à travers l'au moins une surface électriquement conductrice (22) ;
dans lequel le support (12) de l'au moins une électrode (10) est essentiellement longitudinal,
dans lequel l'au moins une électrode (10) comprend en outre des moyens conducteurs (15) configurés pour relier électriquement les moyens d'enregistrement (14) sur l'extrémité proximale (124) avec l'extrémité distale (128) du support (12) ; et
dans lequel la monture (20) est configurée pour se connecter électriquement avec les moyens conducteurs (15) sur l'extrémité distale (128) de l'au moins une électrode (10) à travers l'au moins une surface électriquement conductrice (22) et traiter le signal électrique enregistrée par les moyens d'enregistrement (14) à travers les moyens de traitement (24).

2. Système selon la revendication 1, dans lequel les moyens d'enregistrement (14) sont prévus sur le support (12) par des procédés d'impression ou de gravure.

3. Système selon l'une quelconque des revendications 1 ou 2, dans lequel le support (12) comprend des polymères synthétiques ou naturels.

4. Système selon la revendication 3, dans lequel le support (12) comprend du polyéthylène-glycol (PEG), du poly(téréphtalate d'éthylène), du polyester, de la polyamide.

5. Système selon la revendication 4, dans lequel le support (12) comprend du Biofibre CE0373.

6. Système selon l'une quelconque des revendications 1 à 5, dans lequel au moins le nombre minimum d'éléments n parmi l'au moins une électrode (10) et l'au moins une surface électriquement conductrice (22) sont configurés pour être spatialement appariés un à un, de telle sorte que chacune des n surfaces électriquement conductrices (22) est placée sur la face interne (20a) de la monture (20) dans l'emplacement où son électrode (10) appariée est configurée pour être implantée sur la peau (2).

7. Système selon l'une quelconque des revendications 1 à 6, dans lequel l'au moins une électrode (10) comprend en outre des moyens d'amplification (16) pour amplifier préalablement de manière passive le potentiel enregistré par les moyens d'enregistrement (14).

8. Système selon la revendication 7, dans lequel les moyens d'amplification (16) comprennent des couches semiconductrices, conductrices et isolantes prévues sur le support (12).

9. Système selon l'une quelconque des revendications 1 à 8, dans lequel l'au moins une électrode (10) comprend en outre un élément d'identification (18) configuré pour identifier l'électrode, et dans lequel la monture (20) comprend en outre au moins un moyen d'identification (28) attribué à l'au moins une surface électriquement conductrice (22) configurée pour reconnaître l'élément d'identification (18).

10. Système selon l'une quelconque des revendications 1 à 9, dans lequel les moyens d'enregistrement (14) et/ou les moyens conducteurs (15) de l'au moins une électrode (10) comprennent au moins l'un des matériaux suivants : des polymères conducteurs, des matériaux à base de carbone ou des matériaux à base de métal sous forme de revêtements et de films minces.

11. Système selon la revendication 10, dans lequel les polymères conducteurs sont du PEDOT:PSS, de la polyaniline (PANI), des polyphénylènes, du poly(para-phénylène-vinylène), du polypyrrole (PPy) ou des polythiophènes (PTs) et/ou dans lequel les matériaux à base de carbone sont des nanotubes de carbone, des structures de type « buckyball », de l'oxyde de graphène ou du noir de carbone, et/ou dans lequel les matériaux à base de métal sont Ag, AgCl, TiN, Au ou Pt.

12. Système selon l'une quelconque des revendications 1 à 11, dans lequel le système comprend en outre un dispositif sans fil (30) configuré pour se communiquer avec les moyens de traitement (24) de la monture (20).

13. Système selon la revendication 12, dans lequel le dispositif sans fil (30) est configuré pour recevoir des informations sur la qualité de signal de l'au moins une surface électriquement conductrice (22) à partir de la monture (20) et pour déterminer si la monture (20) est correctement placée sur la peau du sujet sur la base des informations reçues.

14. Système selon l'une quelconque des revendications 1 à 13, dans lequel les moyens d'enregistrement (14) sont configurés pour enregistrer un signal électrique associé à l'activité neuronale d'une région du cerveau et dans lequel la monture (20) est une monture pour la tête et le système est conçu pour acquérir des signaux d'activité neuronale.
